# EUROPÄISCHE PATENTSCHRIFT

(11) **EP 2 173 234 B1**
(45) Veröffentlichungstag und Bekanntmachung des Hinweises auf die Patenterteilung: **06.04.2016**
(21) Anmeldenummer: 08785970.8
(22) Anmeldetag: 08.07.2008
(51) Int. Cl.: A61B 3/103

(54) **VORRICHTUNG UND VERFAHREN ZUR BESTIMMUNG DER ERFORDERLICHEN KORREKTUR DER FEHLSICHTIGKEIT EINES AUGES**
APPARATUS AND METHOD FOR DETERMINING THE NECESSARY CORRECTION OF DEFECTIVE VISION OF AN EYE
DISPOSITIF ET PROCÉDÉ DE DÉTERMINATION DE LA CORRECTION NÉCESSAIRE DU DÉFAUT DE VISION D'UN IL

(30) Priorität: 09.07.2007 DE 102007032001
(43) Veröffentlichungstag der Anmeldung: 14.04.2010
(73) Patentinhaber: Carl Zeiss Vision GmbH, 73430 Aalen (DE)
(72) Erfinder: CABEZA-GUILLÉN, Jesús-Miguel, 73434 Aalen (DE); KRATZER, Timo, 73434 Aalen (DE)
(74) Vertreter: Braunger, Dieter
(86) Internationale Anmeldenummer: PCT/EP2008/058853
(87) Internationale Veröffentlichungsnummer: WO 2009/007368

(56) Entgegenhaltungen:
- EP-B- 1 324 689
- WO-A-2004/096014
- US-A1- 2004 169 820
- US-A1- 2005 213 040
- US-A1- 2006 007 397

## Beschreibung

Vorrichtung und Verfahren zur Bestimmung der erforderlichen Korrektur der Fehlsichtigkeit eines Auges

Die Erfindung betrifft ein Verfahren zur Bestimmung der erforderlichen Korrektur der Fehlsichtigkeit eines Auges nach dem Patentanspruch 1 sowie eine entsprechende Anordnung zur Bestimmung der erforderlichen Korrektur der Fehlsichtigkeit eines Auges nach dem Patentanspruch 8. Schließlich betrifft die Erfindung ein Computerprogramm, ein Computerprogrammprodukt und einen Computer zur Ausführung des erfindungsgemäßen Verfahrens.

Das fehlsichtige menschliche Auge weist refraktive Fehler auf, die in erster Näherung in Form von Sphäre, Zylinder und Achse beschrieben werden können. Dabei wird angenommen, dass sich die Fehlsichtigkeit des Auges näherungsweise durch eine Linse mit einer torischen Fläche korrigieren lässt. Diese Näherung ist hinreichend, um eine fehlerhafte Ablenkung von Lichtstrahlen zu korrigieren, welche auf die Pupillenmitte des Auges treffen.

Während es früher üblich war, die refraktiven Fehler des fehlsichtigen menschlichen Auges aus dem subjektiven Seheindruck des Untersuchten zu bestimmen, wenn ihm eine Mehrzahl von Optotypen unterschiedlicher Brechkraft dargeboten werden (subjektive Refraktion), hat man seit einigen Jahren die Möglichkeit, die refraktiven Fehler des Auges zu messen (objektive Refraktion). Es ist möglich, die Brechkraft des Auges über der ganzen Pupille und insbesondere auch in den Randbereichen der Pupille zu messen. Zu den bestimmbaren Fehlern gehören z.B. sphärische Aberration, Koma, Dreiblattfehler, höhere Arten der sphärischen Aberration etc. Die Ermittlung der objektiven Refraktion erfolgt dadurch, dass die Wellenfront eines sich ausbreitenden Lichtbündels bestimmt wird. Die DE 601 21 123 T2 beschreibt die prinzipielle Wirkungsweise eines Wellenfrontrefraktors und gibt einen Überblick über eine Mehrzahl verschiedener Varianten.

Seit einigen Jahren ist es üblich, die refraktiven Fehler bzw. die Abbildungsfehler des menschlichen Auges mittels sogenannter Zernike-Polynome zu beschreiben. Die zentrumsnahen Fehler des Auges, Sphäre, Zylinder und Achse, lassen sich mit Hilfe von Zernike-Polynomen zweiter Ordnung beschreiben. Deshalb spricht man hier auch häufig von Fehlern zweiter Ordnung. Die zentrumsfernen Fehler des Auges lassen sich mit Zernike-Polynomen höherer Ordnung beschreiben. Diese Fehler werden daher im Allgemeinen auch als Fehler höher Ordnung bezeichnet.

Die von einem Wellenfrontrefraktor gewonnene Information kann genutzt werden, um verbesserte Schhilten bzw. Sehkorrekturmethoden zu entwickeln. Das am besten bekannte Beispiel für ein Sehkorrekturverfahren ist die wellenfrontgeführte refraktive Chirurgie. Mit dieser Behandlung wird ein Volumen mit beliebiger Geometrie aus der Hornhautoberfläche abgetragen, um die Refraktionsfehler inklusive solcher höherer Ordnung zu korrigieren.

Eine solche Korrektur ist mit Sehhilfen, wie z.B. einem Brillenglas oder einer Kontaktlinse, nicht bzw. nur bedingt möglich. Die Besonderheit bei einem Brillenglas liegt darin, dass das Auge durch unterschiedliche Stellen des Brillenglases blicken muss. Eine vollständige Korrektur der Fehler höherer Ordnung in einem Brillenglas ist nur für eine bestimmte Blickrichtung möglich. Sobald das Auge in eine andere Richtung blickt, ist die Korrektur der Fehler höherer Ordnung nicht mehr angepasst und verschlechtert den Visus. Weiterhin würde eine vollständige Korrektur der Fehler höherer Ordnung in einem Brillenglas zu unakzeptablen Verzerrungen außerhalb des Bereichs dieser Korrektur führen.

### Trotzdem kann die Wellenfront-Messtechnik zu verbesserten Brillengläsern führen:

Die subjektive Refraktion wird normalerweise unter Tageslichtbedingungen mit kontrastreichen Optotypen durchgeführt. Dies führt zu Refraktionswerten, die für diese Bedingungen, nämlich insbesondere für eine gute Beleuchtung und für einen hohen Kontrast, optimiert sind. Diese Refraktion ist bei vielen Personen nicht für Nachtsehen oder Sehen in der Dämmerung geeignet. Eine Wellenfrontmessung kann im Dunkeln oder unter mydriatischen Bedingungen durchgeführt werden. Dadurch wird Information für eine viel größere Pupille gewonnen, was z.B. die Ermittlung einer objektiven Refraktion (insbesondere die objektive Refraktion zweiter Ordnung) ermöglicht, die auch für mesopische oder skotopische Lichtbedingungen geeignet ist.

Weiterhin ist es bekannt, dass Brillengläser, insbesondere Gleitsichtgläser, intrinsische Aberrationen aufweisen. Diese intrinsischen Aberrationen können zusammen mit der gemessenen Wellenfront des Auges kombiniert werden, um verbesserte Brillengläser zu berechnen und herzustellen. Diese Brillengläser können für mindestens eine bestimmte Blickrichtung eine zumindest partielle Korrektur der Aberrationen höherer Ordnung des Systems Auge-Brillenglas ermöglichen.

Die Ermittlung einer verbesserten Refraktion zweiter oder höherer Ordnung aus der Wellenfrontmessung ist aus dem Stand der Technik in einer Vielzahl an Varianten bekannt. Aus der US 7,029,119 B2 ist es bekannt, die Refraktion zweiter Ordnung aus den gemittelten Hauptkrümmungen der Wellenfront abzuleiten.

In der EP 1 324 689 B1 wird z.B. ein System zum Bestimmen einer Korrektur zum Korrigieren von Aberrationen in einem Auge eines Patienten beschrieben. Das System umfasst eine Rechenvorrichtung, um die Korrektur von den Datensignalen derart zu bestimmen, dass, wenn die Korrektur auf das Auge angewandt wird, eine Bildqualität-Metrik auf einer Bildebene des Auges objektiv optimiert wird. Die Recheneinrichtung definiert in einem ersten Schritt einen Suchraum (d.h. Werte, welche die Koeffizienten annehmen können), der mehrere Sätze von Koeffizienten (z.B. Sphäre, Zylinder, Achse oder die entsprechenden Zernike-Koeffizienten) umfasst. In einem zweiten Schritt wird die vorher ausgewählte Bildqualität-Metrik (z.B. Strehl-Verhältnis, Varianz der Punktbildverwaschungsfunktion, innerhalb des Airy-Scheibchens eingeschlossene Energie der Punktbildverwaschungsfunktion, etc.) für jeden der Sätze von Koeffizienten in dem Suchraum (d.h. die entsprechenden dioptrischen Werte für Defokus und Astigmatismus sowie die entsprechende Achslage) berechnet. In einem dritten Schritt wird der optimale Wert der Bildqualität-Metrik aus allen Werten der Bildqualität-Metrik, die in dem zweiten Schritt berechnet wurden, ausgewählt und in einem vierten Schritt wird die Korrektur in Übereinstimmung mit einem der mehreren Sätze von Koeffizienten, für den der optimale Wert der Bildqualität-Metrik in dem dritten Schritt berechnet wurde, bestimmt.

L.N. Thibos et al. beschreiben in ihrem Aufsatz "Accuracy and precision of objective refraction from wavefront aberrations", welcher im Journal of Vision (2004) 4, 329-351 am 23. April 2004 veröffentlicht wurde, eine Vielzahl weiterer objektiver Methoden zur Bestimmung der Refraktion aus einer Wellenfrontmessung.

Die vorstehend angegebenen Methoden zur Bestimmung der subjektiven oder objektiven Refraktion vernachlässigen die Physiologie des Auges. Das Auge ist kein statisches System wie die klassischen optischen Systeme; es besitzt die Fähigkeit der Akkommodation. Im Akkommodationsprozess ändert die menschliche Linse sowohl seine Form als auch seine Position, um die Brechkraft des Auges insgesamt zu ändern. Der Akkommodationsprozess ist ein kontinuierlicher Prozess, wobei das System Auge-Gehirn ständig nach Stimuli sucht, um ständig das beste Bild zu generieren. Dies bedeutet dass die gesamte Brechkraft des Auges hochfrequenten Änderungen unterworfen ist. Weiterhin ist es bekannt, dass sich auch die Aberrationsstruktur des Auges mit der Akkommodation des Auges ändert. Insbesondere die sphärische Aberration wird mit der Akkommodation im Durchschnitt negativer. Eine dem objektiven oder subjektiven Refraktionswert entsprechende Brille wird daher vom Träger häufig nicht als optimal empfunden.

Die Aufgabe der Erfindung besteht daher darin, ein Verfahren und eine Anordnung zur Bestimmung der erforderlichen Korrektur der Fehlsichtigkeit eines Auges bereitzustellen, die die Physiologie des Auges auf Basis beispielsweise einer Messung der Wellenfront des Auges in der Berechnung einer Sehkorrektur berücksichtigen. Unter optischer Korrektur versteht man hierbei insbesondere die Stärke einer Brillenlinse oder einer Kontaktlinse aber auch das Ausmaß einer örtlichen Abtragung natürlicher Bestandteile des fehlsichtigen Auges.

Diese Aufgabe wird durch ein Verfahren zur Bestimmung der erforderlichen Korrektur der Fehlsichtigkeit eines Auges mit den Merkmalen des Patentanspruchs 1 sowie eine Anordnung zur Bestimmung der erforderlichen Korrektur der Fehlsichtigkeit eines Auges mit den Merkmalen des Patentanspruchs 8 gelöst.

Vorteilhafte Ausführungen und Weiterbildungen der Erfindung sind in den Unteransprüchen angegeben.

Gemäß dem Hauptanspruch setzt das Verfahren zur Bestimmung der erforderlichen optischen Korrektur der Fehlsichtigkeit eines Auges voraus, dass die optische Korrektur rechnerisch innerhalb eines Ziel- oder Suchraums variiert wird. Dieser Ziel- oder Suchraum muss dabei nicht vorbekannt sein. Es ist möglich, dass die optische Korrektur so lange variiert wird, bis ein Abbruchkriterium erfüllt ist. Ein Abbruchkriterium kann beispielsweise das Erreichen eines Optimums oder eines Werts sehr nahe an diesem Optimum eines nachfolgend angegebenen Zielkriteriums sein.

Gemäß der Erfindung wird nun innerhalb des Zielraums gerade die optische Korrektur als erforderliche optische Korrektur ausgewählt, bei der die Kaustik eine optische Korrektur und Auge transmittierenden Lichtstrahls im Bereich der Netzhaut des Auges vorgegebenen Anforderungen genügt. Anders ausgedrückt bedeutet dies, dass die optische Korrektur ausgewählt wird, bei der die Qualität oder Qualitäten, insbesondere des Volumens, der Kaustik eines durch die optische Korrektur und das Auge propagierenden Lichtstrahls in der räumlichen Umgebung der Netzhaut des Auges vorbestimmten Anforderungen genügt. Es findet also eine Art Vergleich zwischen der Kaustik in der Umgebung der Netzhaut des Auges mit einer Ziel- oder Sollkaustik statt, wobei die Ziel- oder Sollkaustik nicht zwangsläufig stets erreicht zu werden braucht, sondern dieser gegebenenfalls auch nur sehr nahe kommen kann. Unter Kaustik versteht man die mehr oder weniger enge Einschnürung eines Strahlenbündels, die anstelle eines Bildpunkts entsteht, welches das von einem Objektpunkt ausgehende Strahlenbündel infolge von Abbildungsfehlern zeigt, bevor es wieder auseinanderläuft.

Diese geforderten Anforderungen können darin bestehen, dass eine die Qualität der Kaustik beschreibende Metrik einen Optimalwert (Maximum) erreicht, einen gewissen Schwellwert überschreitet oder innerhalb eines Bereichs um den Optimalwert liegt. Dieses Verfahren unterscheidet sich von dem aus dem Stand der Technik bekannten, bei dem die optische Korrektur derart gewählt wird, dass die Refraktion zweiter Ordnung nur für eine Bildebene des Auges optimiert ist.

Die nach dem entsprechenden Anspruch 8 erfindungsgemäß ausgestaltete Anordnung umfasst eine Analyseeinrichtung, um die optische Korrektur innerhalb des Suchraum als erforderliche optische Korrektur auszuwählen, bei der die Kaustik eines durch optische Korrektur und Auge hindurch tretenden Lichtstrahls im Bereich der Netzhaut des Auges den o.a. beschriebenen vorgegebenen Anforderungen genügt.

Konkret umfasst das erfindungsgemäße Verfahren zur Bestimmung der erforderlichen optischen Korrektur der Fehlsichtigkeit eines Auges nach dem Anspruch 2 folgende Verfahrensschritte:

In einem ersten Schritt werden die refraktiven Eigenschaften des Auges bereitgestellt. Das Auge befindet sich dabei vorzugsweise in einem vorgegebenen Akkomodationszustand. Es hat sich als günstig herausgestellt, wenn das Auge auf unendlich fokussiert ist, d.h. wenn die Sehstrahlen der Augen nicht auf einen Punkt in der Nähe konvergieren.

Die refraktiven Eigenschaften des fehlsichtigen Auges können z.B. vorab durch Messung der Wellenfront des zu korrigierenden Auges bestimmt werden. Bei diesem Vorgang spricht man in der englischen Fachsprache auch davon, dass eine "wavefront aberration map" erzeugt wird. Konkret kann eine derartige Wellenfrontmessung mittels des sogenannten Shack-Hartmann Verfahrens oder mittels des sogenannten Tscherning-Verfahrens durchgeführt werden. Bei diesen Verfahren geht man von der Projektion eines Lichtstrahles (Hartmann-Shack) oder eines Lichtpunktmusters (Tscherning) auf die Netzhaut aus. Der Verlauf der reflektierten Strahlen im optischen System unter Wellenfrontbedingungen wird verfolgt. Richtungsänderungen der Strahlen, beziehungsweise eine vom Originalmuster abweichende Abbildung nach Verlassen des optischen Systems werden dargestellt. Die Abweichung des Verlaufs dieser Wellenfront vom idealen Fall wird als Aberration bezeichnet und kann mit einem Aberrometer gemessen werden. Das Hartmann-Shack-Verfahren bedient sich zur Bildaufnahme im allgemeinen einer CCD-Kamera. Einzelheiten zu diesen Verfahren entnimmt man beispielsweise der Dissertation von G.M. Spitziberger "Änderung der optischen Aberrationen des menschlichen Auges durch laser in situ keratomileusis" aus dem Jahr 2004.

Anstelle einer Wellenfrontanalyse der o.a. Art kann auch die sogenannte Ray-Tracing-Methode zur Bestimmung der Brechkraftfehler des fehlsichtigen Auges herangezogen werden. Bei diesem Verfahren wird ein sehr feiner Laserstrahl durch die Pupille des Auges auf die Netzhaut gescannt. Jeder Laserpunkt kann als Reflex auf der Makula identifiziert werden. Die Lage und Form dieser Abbildung auf der Makula erlaubt Aussagen über die Refraktion und Visusqualität.

Die refraktiven Eigenschaften des (menschlichen oder tierischen) Auges können schließlich auch durch Messung der Tomographie des Auges bestimmt werden. Konkret werden die Geometrien der optisch wirksamen Flächen des Auges gemessen; evtl. inklusive der Brechungsindizes der einzelnen Medien.

In einem zweiten Schritt werden Größen von die optische Korrektur beschreibenden Parametersätzen bestimmt. Diese Größen können z.B. aus Sphäre, Zylinder und Achse bestehen bzw. diese umfassen. Es ist auch möglich, dass die Größen sogenannte Flächenbeschreibungen, bzw. Untermengen dieser Flächenbeschreibungen wie Splines, Taylor-Entwicklungen oder Zemike-Entwicklungen bzw. einzelne Koeffizienten dieser Entwicklungen umfassen. Es ist auch möglich, dass die Größen der die optische Korrektur beschreibenden Parametersätze Hauptkrümmungsradien umfassen.

Die Rechnertechnologie erlaubt es, als die optische Korrektur beschreibenden Größen die Koeffizienten einer für die Beschreibung der optischen Korrektur geeigneten algebraischen Basis- oder Flächendarstellung wie z.B. Spline-, Zernike- oder Taylor-Koeffizienten zu verwenden.

In einem dritten Schritt ist vorgesehen, ein geeignetes Verfahren zur Bestimmung eines Zielparametersatzes aus den Parametersätzen, bereitzustellen, der für die in dem ersten Schritt bereitgestellten bzw. bestimmten refraktiven Eigenschaften des Auges die optimale optische Korrektur liefert, die den vorgegebenen Anforderungen genügt. Nachfolgend wird diese optische Korrektur als optische Zielkorrektur oder für den nachfolgend beschriebenen Fall eines Optimierungsverfahrens als optimale optische Korrektur bezeichnet.

Als geeignet haben sich z.B. ein Newton-Raphson Verfahren, ein Hill-Climbing Verfahren oder ein "Alles Ausprobier Verfahren", bei dem innerhalb eines Suchraums von Parametersätzen alle darin befindlichen Parametersätze auf die vorgegebenen Anforderungen, insbesondere auf ein Optimum hin überprüft werden, erwiesen.

Es sei darauf hingewiesen, dass die vorstehend angegebenen drei Schritte keine zeitliche Reihenfolge definieren, vielmehr ist es unerheblich, welcher der drei Schritte zuerst und in welcher Folge die entsprechenden Informationen bereitgestellt werden. Es kommt allein darauf an, dass die Informationen für den nachfolgenden Vorgang zur Bestimmung der optischen Zielkorrektur, insbesondere der optimalen optischen Korrektur zur Verfügung stehen.

In einem (nachfolgenden) vierten Schritt werden nun mindestens zwei Untermetriken in verschiedenen zugeordneten Propagationsstadien von Licht durch das Auge und optische Korrektur umfassende optische System für einen der Parametersätze bestimmt. Anders ausgedrückt, das Licht tritt durch das optische System Auge/Korrektur. Man betrachtet nun die jeweils durch eine Qualitätsmetrik (Untermetrik) ausgedrückte Abweichung des Lichtstrahls gegenüber dem Idealfall, wenn dieser unterschiedlich weit durch das Auge bzw. das System Auge/Korrektur hindurchgetreten (propagiert) ist. Ebenso ist eine Propagation in umgekehrter Richtung, also vom System Auge/Korrektur in Richtung Objekt denkbar. Diese Propagation ist dabei nicht auf eine feste Richtung durch das System Auge/Korrektur festgelegt, sondern kann für beliebig viele Richtungen (in allgemeinen Blickrichtungen) durchgeführt werden.

In der Literatur (z.B. L.N. Thibos et al a.a.O.) wird häufig zwischen Pupillenmetriken (engl.: pupil-plane metrics) und Bildqualitätsmetriken (engl.: image-plane metrics) unterschieden. Es ist für den Fachmann selbstverständlich, dass er grundsätzlich beide Arten von Qualitätsmetriken als Untermetriken verwenden kann.

So können diese Untermetriken beispielsweise Strahlqualitätsmetriken, wie z.B. Metriken, die das Strehl-Verhältnis oder die innerhalb des Airy-Scheibchens eingeschlossene Energie der Punktbildverwaschungsfunktion sein. Es ist auch möglich, dass die Untermetriken geometrische Metriken, wie z.B. solche, die die mittlere Krümmung der Wellenfront berücksichtigen, sind.

Es ist auch möglich, die neuronale Signalverarbeitung des von dem menschlichen Auge erfassten Bildes zu berücksichtigen, wie dies z.B. auch in L.N. Thibos et al auf Seite 330, rechte Spalte, Mitte unter Hinweis auf diverse weitere Literaturstellen diskutiert wird.

In einem nachfolgenden fünften Schritt wird eine (insbesondere die Qualität der Kaustik reflektierende) Gesamtmetrik (Kaustikmetrik) aus einer gewichteten Summe der zuvor bestimmten Untermetriken bestimmt. Es ist möglich, dass alle Untermetriken bei der Bestimmung der Gesamtmetrik (Kaustikmetrik) gleich gewichtet werden. Es hat sich jedoch als günstig herausgestellt, wenn eine Untermetrik eines Vorzugspropagationsstadiums stärker gewichtet wird als die Untermetriken in den Propagationsstadien vor und/oder hinter diesem Vorzugspropagationsstadium. Geht man beispielsweise von Untermetriken aus, die die Bildqualität in unterschiedlichen Ebenen berücksichtigen, so würde z.B. bevorzugt die Untermetrik für das Bild auf der Retina (entspricht der Untermetrik im Vorzugspropagationsstadium) stärker gewichtet als die Untermetrik für ein Bild vor oder hinter der Netzhaut des Auges. Die Gewichtung könnte z.B. 60/40 betragen.

Geht man von mehr als zwei oder drei Untermetriken, in unterschiedlichen Propagationsstadien aus, so hat es sich weiter als günstig herausgestellt, wenn die Untermetriken in den Propagationsstadien vor und/oder hinter dem Vorzugspropagationsstadium mit zunehmendem Abstand vom Vorzugspropagationsstadium weniger stark gewichtet werden. Unter beispielhafter Annahme von Untermetriken, die die Bildqualität in unterschiedlichen Ebenen berücksichtigen (siehe oben), würde bevorzugt die Untermetrik für das Bild auf der Retina (entspricht der Untermetrik im Vorzugspropagationsstadium) stärker gewichtet als die Untermetrik für ein Bild in einem Abstand von 0,5 dpt vor oder hinter der Netzhaut des Auges. Die Untermetrik für ein Bild in einem Abstand von 0,5 dpt vor oder hinter der Netzhaut würde wiederum stärker gewichtet werden als die Untermetrik für ein Bild in einem Abstand von 1 dpt von der Retina. Die Gewichtung könnte z.B. 50/30/20 betragen, wenn vor der Retina keine Untermetrik berücksichtigt wird, hinter der Retina jedoch zwei weitere Untermetriken in verschiedenen Bildebenen in die Kalkulation einfließen. Ebenso ist es möglich bei Propagation in Objektrichtung als Vorzugspropagationsstadium die Objektebene zu nehmen.

In einer weiteren Ausführungsform hat es sich als günstig herausgestellt, wenn statt einzelner diskreter Untermetriken die Intensitätsverteilung im dreidimensionalen Raum des Strahlbündels mit Hilfe eines modifizierten Nijboer-Zernike-Formalismus berechnet wird (Kontinuum von Untermetriken) und die Energiedichte entlang dieser Intensitätsverteilung als Parameter für die Bestimmung des Zielparametersatzes, insbes. zur Optimierung des optimalen Parametersatzes verwendet wird.

In einem hierauf folgenden sechsten Schritt werden die Schritte vier und fünf für sämtliche Parametersätze durchgeführt, die zur Bestimmung des Zielparametersatzes nach dem in Schritt drei bereitgestellten Verfahren erforderlich sind.

Im siebten Schritt wird der Zielparametersatz aus den Parametersätzen für die die Schritte vier und fünf durchgeführt wurden, ausgewählt, der die Zielgesamtmetrik (Zielkaustikmetrik) liefert, die den angegebenen Anforderungen genügt. Beispielsweise ist die optimale Gesamtmetrik im Allgemeinen die Metrik mit dem maximalen (oder hiervon geringfügig abweichenden) Wert.

Diese Verfahrensschritte können für verschiedene Akkommodationszustände des Auges durchgeführt werden. Ist dies der Fall so wird in einem nachfolgenden Schritt ein finaler Zielparametersatz berechnet, der die finale Zielgesamtmetrik (z.B. finale optimale Gesamtmetrik) liefert aus Wichtungen aller zuvor bestimmten Zielgesamtmetriken (z.B. optimalen Gesamtmetriken) für die unterschiedlichen Akkommodationszustände des Auges.

In einem achten Schritt wird die erforderliche optische Korrektur aus dem in Schritt sieben oder ggf. den für verschiedene Akkommodationszustände des Auges ausgewählten (finalen) Zielparametersatz (z.B. optimalen (finalen) Parametersatz) bestimmt.

Die erfindungsgemäße Anordnung zur Bestimmung der erforderlichen optischen Korrektur der Fehlsichtigkeit eines Auges umfasst eine Eingabeeinrichtung zur Bereitstellung der refraktiven Eigenschaften des Auges sowie eine Analyseeinrichtung. Die Analyseeinrichtung ist dazu vorgesehen zunächst mindestens zwei Untermetriken in verschiedenen zugeordneten Propagationsstadien eines Lichtstrahls durch das Auge und Korrektur umfassende optische System für einen Parametersatz der die optische Korrektur beschreibenden Größen zu bestimmen. Aus einer gewichteten Summe der Untermetriken bestimmt die Analyseeinrichtung dann eine Gesamtmetrik. Dieser Vorgang der Bestimmung von Untermetriken und nachfolgende Berechnung einer Gesamtmetrik wird von der Analyseeinrichtung für weitere Parametersätze der die optische Korrektur beschreibenden Größen wiederholt, die zur Bestimmung eines Zielparametersatzes (z.B. optimalen Parametersatzes) erforderlich sind. Die Analyseeinrichtung ist weiter dazu eingerichtet, den Zielparametersatz (z.B. optimalen Parametersatz) aus den Parametersätzen für die der Vorgang der Bestimmung von Untermetriken und nachfolgende Berechnung einer Gesamtmetrik durchgeführt wurde, auszuwählen, der die Zielgesamtmetrik (z.B. optimale Gesamtmetrik) liefert. Die Analyseeinrichtung ist weiter ausgebildet, die erforderliche optische Korrektur aus dem im vorigen Schritt ausgewählten Zielparametersatz (z.B. optimalen Parametersatz) zu bestimmen. Vorzugsweise weist die erfindungsgemäße Anordnung eine Ausgabeeinrichtung auf, um die die optische Korrektur definierenden Informationen für einen Benutzer erfassbar auszugeben.

Die Eingabeeinrichtung kann beispielsweise eine Tastatur umfassen, welcher durch eine Wellenfrontmessung bestimmte Informationen über die refraktiven Eigenschaften des Auges zuführbar sind.

Alternativ oder zusätzlich kann die Eingabeeinrichtung mit einer Wellenfrontmesseinrichtung zur Messung der refraktiven Eigenschaften des Auges (Wellenfrontrefraktor) und/oder mit einem Aberrometer nach dem Shack-Hartmann-Prinzip und/oder mit einem Aberrometer für das Tscherning-Verfahren und/oder mit einem Tomographen für das Auge und/oder mit einem nach der Ray-Tracing-Methode arbeitenden Aberrometer über eine geeignete Schnittstelle verbunden sein.

Des Weiteren werden ein Computerprogramm, ein Computerprogrammprodukt und ein Computer zur Ausführung des Computerprogramms vorgeschlagen, um das erfindungsgemäße Verfahren durchzuführen. Das Computerprogramm kann mit Programmcodemitteln ausgestattet sein, um alle Schritte des Verfahrens nach einer der o.a. Varianten durchzuführen. Das Computerprogrammprodukt kann mit Programmcodemitteln ausgestattet sein, die auf einem computerlesbaren Datenträger gespeichert sind, um alle Schritte des Verfahrens nach einer der o.a. Varianten durchzuführen, wenn das Computerrogrammprodukt auf einem Computer ausgeführt wird. Das Computerprogrammprodukt kann z.B. eingereichtet sein, um über das Internet oder vergleichbare Netze die Analyse der Daten unabhängig vom Ort der Datenaufnahme an einem beliebigen Ort vorzunehmen. Das Computerprogramm kann eif einem Datenträger gespeichert sein. Erfindungsgemäß kann ein Computer mit einer Anzeigeeinrichtung und einer Eingabeeinrichtung vorgesehen sein, welcher zur Ausführung des Computerprogramms eingerichtet ist.

Die Erfindung wird nachfolgend anhand der Zeichnung näher beschrieben. Gleiche oder funktionsgleiche Bestandteile sind in allen Figuren mit identischen Bezugszeichen versehen. Es zeigen:
- Figur 1:: ein menschliches fehlsichtiges Auge mit einer Brillenlinse, wobei die Brecheigenschaften der Brillenlinse in konventioneller Weise derart gewählt sind, dass eine Bildqualitätsmetrik auf der Netzhaut des Auges objektiv optimiert wird,
- Figur 2:: ein menschliches fehlsichtiges Auge mit einer Brillenlinse, wobei die Brecheigenschaften der Brillenlinse erfindungsgemäß derart gewählt sind, dass die Kaustik eines in das Auge einfallenden Lichtbündels im Bereich der Netzhaut des Auges objektiv optimiert wird,
- Figur 3:: das menschliche Auge nach der Figur 2 mit schematischer Darstellung der Punktbildverwaschungsfunktion in unterschiedlichen Propargationsstadien des Lichtes.

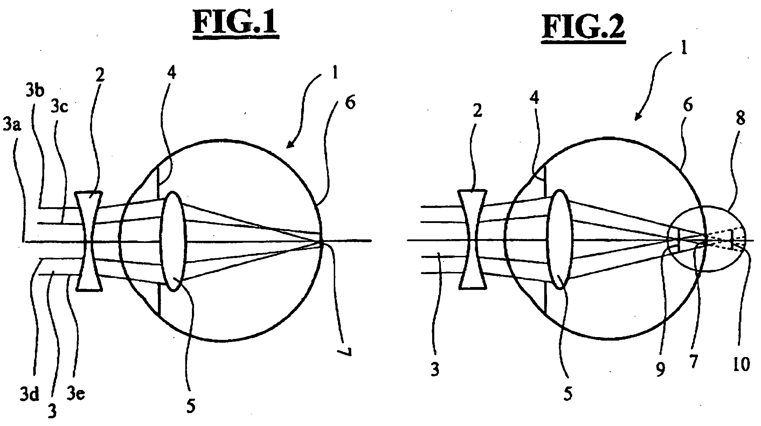

Die Figur 1 zeigt ein fehlsichtiges menschliches Auge 1 mit einer Brillenlinse 2 im Querschnitt. Ein Bündel 3 paralleler Lichtstrahlen 3a, 3b, 3c, 3d, 3e tritt über die Brillenlinse 2 in das Auge 1 ein. Die Iris 4 begrenzt die einfallende Lichtmenge. Die Lichtstrahlen 3a, 3b, 3c, 3d, 3e werden aufgrund der nicht idealen Brechkraft des die Brillenlinse 2 und die Augenlinse 5 umfassenden optischen Systems in nicht idealer Weise auf die Netzhaut (Retina) 6 des Auges 1 abgebildet.

Aus dem Stand der Technik, beispielhaft sei die EP 1 324 689 B1 genannt, ist es bekannt, die Refraktion zweiter Ordnung der Brillenlinse 2 derart zu wählen, dass diese in einer Bildebene, vorzugsweise in der Netzhautebene 7 ein optimales Bild liefert. Dieses Verfahren kann dazu führen, dass die Bildqualität außerhalb dieser Ebene 7 sehr schnell abfällt. Ein derartiger Abfall kann z.B. bei hohen sphärischen Aberrationen sehr schnell auftreten. Für das Auge würde dies eine außerordentliche Anstrengung bedeuten, da der akkommodative Zustand des Auges sehr genau gehalten werden muss, um eine gute Bildqualität zu erhalten.

Dieses Problem wird erfindungsgemäß dadurch gelöst, dass die Kaustik 8 des Lichtbündels 3 um die Bildebene 7, d.h. Ebene auf der Retina 6, auf die das Lichtbündel 3 abgebildet wird, optimiert wird (vgl. Figur 2). Durch dieses Vorgehen könnte z.B. die Schärfentiefe der Abbildung erhöht werden. Gleichzeitig könnte die Bildqualität auf der Bildebene 7 selbst eventuell geringfügig schlechter werden als das dort erreichbare Optimum. Dafür sorgt diese Maßnahme aber für einen angenehmeren und beschwerdefreieren Seheindruck, weil Fluktuationen des Auges eliminiert werden.

### Die Optimierung der Kaustik kann beispielsweise wie folgt durchgeführt werden:

Zunächst wird eine sogenannte "wavefront aberration map" des sich in einem vorgegebenen Akkommodationszustand befindenden Auges bestimmt. Anders ausgedrückt erfolgt eine Wellenfrontmessung für eine bestimmte vorgegebene Pupillenfläche.

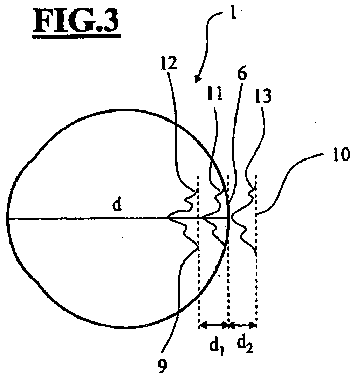

Dann wird ein Suchraum von Sätzen die optische Korrektur beschreibender Größen, wie z.B. Sphäre, Zylinder, Achse, bestimmt. Weiter wird eine ein Maß für die aus der Anwendung der optischen Korrektur 2 auf das Auge 1 resultierende Bildqualität in der Bildebene 7 des Auges 1 darstellende Bildqualitätsmetrik für jeden Satz innerhalb dieses Suchraums bestimmt. In gleicher Weise werden in einer Ebene 9 vor der Bildebene 7 des Auges 1 und in einer Ebene 10 hinter der Bildebene 7 des Auges 1die Bildqualitätsmetriken für jeden Satz innerhalb des Suchraums bestimmt. Die Ebene 9 kann z.B. d₁=½ dpt (= etwa 0,3mm) vor der Retina 6, die Ebene 10 etwa d₂= - ½ dpt (= etwa 0,3mm) hinter der Retina 6 liegen. Das Standardauge weist eine Abmessung d von etwa 43dpt (= etwa 2,47cm) auf. Die Figur 3 zeigt zur Verdeutlichung dieses Sachverhalts die Punktstrahlenfunktionen 11, 12, 13 in den vorstehend angegebenen Ebenen 7, 9, 10 eines menschlichen Auges 1.

Durch entsprechende Gewichtung der Bildqualitätsmetriken in den unterschiedlichen Ebenen 7, 9, 10 wird für jeden Parametersatz innerhalb des Suchraums eine ein Maß für die Kaustik im Bereich der drei Ebenen 7, 9, 10 darstellende Kaustikmetrik berechnet.

Diese Kaustikmetrik ist also ein Maß für die Güte der Kaustik für den jeweiligen Parametersatz.

Aus allen berechneten Gesamtmetriken, deren Anzahl der Zahl der Sätze innerhalb des Suchraums entspricht, wird nun die optimale Gesamtmetrik, d.h. die Kaustik mit der höchsten Güte, ausgewählt. Die erforderliche optische Korrektur, d.h. die Brechkraftverteilung der Brillenlinse 2 oder die Wellenfront der Brillenlinse 2, wird schließlich unter Berücksichtigung des Satzes aus dem die ausgewählte optimale Gesamtmetrik resultiert, bestimmt.

Anstelle der Bestimmung einer "wave front aberration map" für einen einzigen Akkommodationszustand eines Auges können auch "aberration maps" für mehrere Akkommodationszustände bestimmt werden und die o.a. Prozedur für sämtliche Akkommodationszustände durchgeführt werden. Der vorstehend beschriebene Optimierprozess kann also verbessert werden, indem nicht nur die Wellenfront des Auges für die Ferne verwendet wird. Wenn man über Wellenfrontmessung spricht, bezieht man sich normalerweise auf die Wellenfront des Auges, welches auf unendlich akkommodiert. Es ist aber auch möglich, die Wellenfront des Auges in unterschiedlichen Akkommodationszuständen zu messen. Daraus entsteht ein Satz von Wellenfronten des Auges. Der obige Optimierprozess kann für die unterschiedlichen Wellenfronten für unterschiedliche Akkommodationszustände wiederholt werden. Daraus entsteht eine optische Korrektur, die neben der Ferneoptimierung gleichzeitig eine Optimierung der Nähe beinhaltet.

Weiterhin ist es z.B. möglich, nur die Bildqualitatsmetriken zweier Ebenen vor und hinter der Retinaebene zu berücksichtigen und hieraus eine Gesamtmetrik abzuleiten.

Anstelle der Bestimmung von mehreren Bildqualitätsmetriken in unterschiedlichen Schnittebenen und der Berechnung eines eine Gesamtmetrik darstellenden Mittelwerts kann die Qualität der Kaustik um die Bildebene z.B. auch durch Ray-Tracing unter Annahme eines geeigneten Augenmodels ermittelt werden.

Für die Auswertung der Qualität der Kaustik können unterschiedliche Metriken angewendet werden, z.B. der Durchmesser der Kaustik der mehr als einen gewissen Anteil der Energie einschließt, oder der Energieanteil, der in einem gewissen Bereich um die optische Achse anfällt, oder andere Metriken.

In dem obigen Optimierprozess sind auch die intrinsischen Aberrationen des Brillenglases berücksichtigt.

Die nachfolgend aufgeführten Gegenstände sind im Sinne von Klauseln formuliert und stellen weitere Verfahren, Anordnungen Computerprogramme und Computerprogrammprodukte dar, die nicht Gegenstand der Erfindung sind.
A. Verfahren zur Bestimmung der erforderlichen optischen Korrektur (2) der Fehlsichtigkeit eines Auges (1) bei dem die optische Korrektur (2) rechnerisch innerhalb eines Zielraums variiert wird, dadurch gekennzeichnet, dass die optische Korrektur (2) innerhalb des Zielraums als erforderliche optische Korrektur (2) ausgewählt wird, bei der die Kaustik (8) eines durch die optische Korrektur (2) und das Auge (1) hindurch tretenden Lichtstrahls im Bereich der Netzhaut (6) des Auges (1) vorgegebenen Anforderungen genügt.
B. Verfahren zur Bestimmung der erforderlichen optischen Korrektur (2) der Fehlsichtigkeit eines Auges (1), insbesondere nach Klausel A, mit folgenden Verfahrensschritten:
   a) Bereitstellung der refraktiven Eigenschaften des Auges (1),
   b) Bereitstellen von Größen von die optische Korrektur (2) beschreibenden Parametersätzen,
   c) Bereitstellen eines Verfahrens zur Bestimmung eines Zielparametersatzes aus den die optische Korrektur (2) beschreibenden Parametersätzen, der für die in Schritt a) bereitgestellten refraktiven Eigenschaften des Auges (1) die optische Zielkorrektur (2) liefert,
   d) Bestimmung von mindestens zwei Untermetriken in verschiedenen zugeordneten Propagationsstadien (7, 9, 10) eines Lichtstrahls (3) durch das Auge und Korrektur umfassende optische System für einen der Parametersätze,
   e) Bestimmung einer Gesamtmetrik aus einer gewichteten Summe der Untermetriken,
   f) Durchführung der Schritte d) und e) für die Parametersätze, die zur Bestimmung des Zielparametersatzes nach dem in Schritt c) bereitgestellten Verfahren erforderlich sind,
   g) Auswahl des Zielparametersatzes, der die Zielgesamtmetrik liefert, aus den Parametersätzen für die die Schritte d) und e) durchgeführt wurden,
   h) Bestimmung der erforderlichen optischen Korrektur (2) unter Berücksichtigung des in Schritt g) ausgewählten Zielparametersatzes.
C. Verfahren zur Bestimmung der erforderlichen optischen Korrektur (2) der Fehlsichtigkeit eines Auges (1), insbesondere nach Klausel A, mit folgenden Verfahrensschritten:
   a) Bereitstellung der refraktiven Eigenschaften des Auges (1),
   b) Bereitstellen von Größen von die optische Korrektur (2) beschreibenden Parametersätzen,
   c) Bereitstellen eines Verfahrens zur Bestimmung eines Zielparametersatzes aus den die optische Korrektur (2) beschreibenden Parametersätzen, der für die in Schritt a) bereitgestellten refraktiven Eigenschaften des Auges (1) eine optische Zielkorrektur (2) liefert,
   d) Bestimmung von mindestens einer Metrik, die die Energiedichte der Intensitätsverteilung im 3-dimensionalen Raum eines Lichtsbündels durch das Auge und optische Korrektur umfassenden optischen Systems charakterisiert,
   e) Durchführung des Schrittes d) für die Parametersätze, die zur Bestimmung des Zielparametersatzes nach dem in Schritt c) bereitgestellten Verfahren erforderlich sind,
   f) Auswahl des Zielparametersatzes, der die Zielgesamtmetrik liefert, aus den Parametersätzen für die der Schritt d) durchgeführt wurde,
   g) Bestimmung der erforderlichen optischen Korrektur (2) unter Berücksichtigung des in Schritt f) ausgewählten Zielparametersatzes.
D. Verfahren nach Klausel B oder C, wobei die refraktiven Eigenschaften des Auges (1) durch Messung der Wellenfront, insbesondere mittels des Shack-Hartmann-Verfahrens und/oder mittels des Tscherning-Verfahrens und/oder mittels der Ray-Tracing-Methode bestimmt werden.
E. Verfahren nach Klausel B oder C, wobei die refraktiven Eigenschaften des Auges (1) durch Messung der Tomographie des Auges (1) bestimmt werden.
F. Verfahren nach einer der Klauseln B bis E, wobei die Größen der die optische Korrektur (2) beschreibenden Parametersätze Sphäre, Zylinder und Achse umfassen.
G. Verfahren nach einer der Klauseln B bis E, wobei die Größen der die optische Korrektur (2) beschreibenden Parametersätze Hauptkrümmungsradien umfassen.
H. Verfahren nach einer der Klauseln B bis E, wobei die Größen der die optische Korrektur (2) beschreibenden Parametersätze aus den Koeffizienten einer für die Beschreibung der optischen Korrektur geeigneten algebraischen Basis bestehen.
I. Verfahren nach Klausel H, wobei die algebraische Basis Zernike-Koeffizienten oder Taylor-Koeffizienten umfasst.
J. Verfahren nach einer der Klauseln B bis I, wobei das Verfahrens zur Bestimmung eines Zielparametersatzes aus den die optische Korrektur (2) beschreibenden Parametersätzen ein Newton-Raphson Verfahren oder ein Hill-Climbing Verfahren oder ein Verfahren ist, bei dem innerhalb eines Suchraums von Parametersätzen alle darin befindlichen Parametersätze auf ein Optimum hin überprüft werden.
K. Verfahren nach einer der Klauseln B und D bis J, wobei die Untermetriken Strahlqualitätsmetriken sind.
L. Verfahren nach Klausel K, wobei die Strahlqualitätsmetriken das Strehl-Verhältnis und/oder die innerhalb eines Querschnitts eingeschlossene Energie ist, durch den der Lichtstrahl hindurch tritt.
M. Verfahren nach einer der Klauseln B und D bis L, wobei die Untermetriken geometrische Metriken sind.
N. Verfahren nach Klausel M, wobei die geometrischen Metriken die mittlere Krümmung der Wellenfront umfassen.
O. Verfahren nach einer der Klauseln B und D bis N, wobei alle Untermetriken gleich gewichtet werden.
P. Verfahren nach einer der Klauseln B und D bis N, wobei die Untermetrik in einem Vorzugspropagationsstadium (7) stärker gewichtet wird als die Untermetriken in den Propagationsstadien (9, 10) vor und/oder hinter dem Vorzugspropagationsstadium (7).
Q. Verfahren nach Klausel P, wobei die Untermetriken in den Propagationsstadien (9, 10) vor und/oder hinter dem Vorzugspropagationsstadium (7) mit zunehmendem Abstand vom Vorzugspropagationsstadium (7) geringer gewichtet werden.
R. Verfahren nach einer der Klauseln B bis Q, wobei die Zielgesamtmetrik die Gesamtmetrik mit dem maximalen Wert oder einem von diesem geringfügig abweichenden Wert ist.
S. Verfahren nach einer der Klauseln B bis R, wobei folgende weitere Verfahrensschritte
   i) Durchführen der Verfahrensschritte a) bis h) für verschiedene Akkommodationszustände des Auges (1),
   j) Auswahl des finalen Zielparametersatzes, der die finale Zielgesamtmetrik liefert aus allen in Schritt i) bestimmten Zielgesamtmetriken für die unterschiedlichen Akkommodationszustände des Auges (1),
   k) Bestimmung der erforderlichen optischen Korrektur unter Berücksichtigung des in Schritt j) ausgewählten finalen Zielparametersatzes.
T. Anordnung zur Bestimmung der erforderlichen optischen Korrektur (2) der Fehlsichtigkeit eines Auges (1), welche rechnerisch die optische Korrektur (2) innerhalb eines Zielraums variiert, dadurch gekennzeichnet, dass eine Analyseeinrichtung vorgesehen ist, welche die optische Korrektur (2) innerhalb des Zielraums als erforderliche optische Korrektur (2) auswählt, bei der die Kaustik eines durch die optische Korrektur (2) und das Auge (1) hindurch tretenden Lichtstrahls (3) im Bereich der Netzhaut (6) des Auges (1) vorgegebenen Anforderungen genügt.
U. Anordnung zur Bestimmung der erforderlichen optischen Korrektur (2) der Fehlsichtigkeit eines Auges (1), insbesondere nach Klausel T, mit
   - einer Eingabeeinrichtung zur Bereitstellung der refraktiven Eigenschaften des Auges (1),
   - einer Analyseeinrichtung, welche
      i) mindestens zwei Untermetriken in verschiedenen zugeordneten Propagationsstadien (7, 9, 10) eines Lichtstrahls (3) durch das Auge (1) und optische Korrektur (2) umfassende optische System für einen Parametersatz der die optische Korrektur (2) beschreibenden Größen bestimmt,
      ii) aus einer gewichteten Summe der Untermetriken eine Gesamtmetrik bestimmt,
      iii) die Schritte i) und ii) für weitere Parametersätze der die optische Korrektur (2) beschreibenden Größen wiederholt, die zur Bestimmung eines Zielparametersatzes erforderlich sind,
      iv) den Zielparametersatz aus den Parametersätzen für die die Schritte i) und ii) durchgeführt wurden, auswählt, der die Zielgesamtmetrik liefert, und v) die erforderliche optische Korrektur (2) unter Berücksichtigung des in Schritt iv) ausgewählten Zielparametersatzes bestimmt.
V. Anordnung zur Bestimmung der erforderlichen optischen Korrektur (2) der Fehlsichtigkeit eines Auges (1), insbesondere nach Klausel T, mit
   - einer Eingabeeinrichtung zur Bereitstellung der refraktiven Eigenschaften des Auges (1)
   - einer Analyseeinrichtung, welche
      i) Größen von die optische Korrektur (2) beschreibenden Parametersätzen bereitstellt,
      ii) ein Verfahren zur Bestimmung eines Zielparametersatzes aus den die optische Korrektur (2) beschreibenden Parametersätzen bereitstellt, der für die in Schritt i) bereitgestellten refraktiven Eigenschaften des Auges (1) eine optische Zielkorrektur (2) liefert,
      iii) mindestens eine Metrik bestimmt, die die Energiedichte der Intensitätsverteilung im 3- dimensionalen Raum eines Lichtbündels durch das Auge (1) und optische Korrektur (2) umfassenden optischen Systems charakterisiert,
      iv) Schritt iii) für die Parametersätze durchführt, die zur Bestimmung des Zielparametersatzes nach dem in Schnitt ii) bereitgestellten Verfahren erforderlich sind,
      v) den Zielparametersatz auswählt, der die Zielmetrik liefert, aus den Parametersätzen für die der Schritt iv) durchgeführt wurde,
      vi) die erforderliche optische Korrektur (2) unter Berücksichtigung des in Schritt v) ausgewählten Zielparametersatzes bestimmt.
W. Anordnung nach Klausel U oder V, wobei die Analyse in der Analyseeinrichtung nah oder fern dem Ort der Datenaufnahme stattfindet.
X. Anordnung nach einer der Klauseln U, V oder W, wobei die Eingabeeinrichtung eine Tastatur umfasst, welcher Informationen über die refraktiven Eigenschaften des Auges (1) zuführbar sind.
Y. Anordnung nach einer der Klauseln U bis X, wobei die Eingabeeinrichtung mit einer Wellenfrontmesseinrichtung zur Messung der refraktiven Eigenschaften des Auges (1) und/oder mit einem Aberrometer nach dem Shack-Hartmann-Prinzip und/oder mit einem Tomographen für das Auge und/oder mit einem Aberrometer für das Tscherning-Verfahren und/oder mit einem Aberrometer nach der Ray-Tracing-Methode verbunden ist.
Z. Computerprogramm mit Programmcodemitteln, um alle Schritte des Verfahrens nach einem der Ansprüche A bis R durchzuführen.
AA. Computerprogrammprodukt mit Programmcodemitteln, die auf einem computerlesbaren Datenträger gespeichert sind, um alle Schritte des Verfahrens nach einem der Ansprüche A bis 1 R durchzuführen, wenn das Programmprodukt auf einem Computer ausgeführt wird.
AB. Computerprogrammprodukt nach Klausel AA, welches über das Internet oder vergleichbare Netze die Analyse der Daten unabhängig vom Ort der Datenaufnahme an einem beliebigen Ort vornimmt.
AC. Datenträger auf dem ein Computerprogramm nach Klausel Z gespeichert ist.
AD. Computer mit einer Anzeigeeinrichtung und einer Eingabeeinrichtung, eingerichtet zur Ausführung des Computerprogramms gemäß Klausel Z.

## Patentansprüche

1. Verfahren zur Bestimmung der erforderlichen optischen Korrektur (2) der Fehlsichtigkeit eines Auges (1) bei dem die optische Korrektur (2) rechnerisch innerhalb eines Zielraums variiert wird,
**dadurch gekennzeichnet, dass**
die Kaustik, nämlich die Einschnürung eines Strahlenbündels, die anstelle eines Bildpunkts entsteht, welche das von einem Objektpunkt angehende Strahlenbündel infolge von Abbildungsfehlern zeigt, bevor es wieder auseinander läuft eines die optische Korrektur und das Auge transmittierenden Lichtstrahls in der räumlichen Umgebung der Netzhaut des Auges mit einer Sollkaustik verglichen wird, wobei die optische Korrektur so lange variiert wird, bis ein Abbruchkriterium erfüllt ist, und dass die optische Korrektur (2) innerhalb des Zielraums als erforderliche optische Korrektur (2) ausgewählt wird, bei der die Kaustik (8) eines durch die optische Korrektur (2) und das Auge (1) hindurch tretenden Lichtstrahls im Bereich der Netzhaut (6) des Auges (1) vorgegebenen Anforderungen genügt, indem sie die Sollkaustik erreicht oder der Sollkaustik sehr nahe kommt.

2. Verfahren zur Bestimmung der erforderlichen optischen Korrektur (2) der Fehlsichtigkeit eines Auges (1), nach Anspruch 1, mit folgenden Verfahrensschritten:
a) Bereitstellung der refraktiven Eigenschaften des Auges (1),
b) Bereitstellen von Größen von die optische Korrektur (2) beschreibenden Parametersätzen innerhalb eines Suchraums,
c) Bereitstellen eines Verfahrens zur Bestimmung eines Zielparametersatzes aus den die optische Korrektur (2) beschreibenden Parametersätzen, der für die in Schritt a) bereitgestellten refraktiven Eigenschaften des Auges (1) die optische Zielkorrektur (2) liefert,
d) Bestimmung von mindestens zwei Untermetriken in verschiedenen zugeordneten Propagationsstadien (7, 9, 10) eines Lichtstrahls (3) durch das Auge und Korrektur umfassende optische System für einen der Parametersätze und die refraktiven Eigenschaften des Auges, wobei die Propagationsstadien dadurch festgelegt sind, wie weit ein Lichtstrahl durch das optische System propagiert ist,
e) Bestimmung einer Gesamtmetrik aus einer gewichteten Summe der Untermetriken,
f) Durchführung der Schritte d) und e) für die Parametersätze, die zur Bestimmung des Zielparametersatzes nach dem in Schritt c) bereitgestellten Verfahren erforderlich sind,
g) Auswahl des Zielparametersatzes, der die Zielgesamtmetrik liefert, aus den Parametersätzen für die die Schritte d) und e) durchgeführt wurden,
h) Bestimmung der erforderlichen optischen Korrektur (2) unter Berücksichtigung des in Schritt g) ausgewählten Zielparametersatzes.

3. Verfahren zur Bestimmung der erforderlichen optischen Korrektur (2) der Fehlsichtigkeit eines Auges (1), nach Anspruch 1, mit folgenden Verfahrensschritten:
a) Bereitstellung der refraktiven Eigenschaften des Auges (1),
b) Bereitstellen von Größen von die optische Korrektur (2) beschreibenden Parametersätzen innerhalb eines Suchraums,
c) Bereitstellen eines Verfahrens zur Bestimmung eines Zielparametersatzes aus den die optische Korrektur (2) beschreibenden Parametersätzen, der für die in Schritt a) bereitgestellten refraktiven Eigenschaften des Auges (1) eine optische Zielkorrektur (2) liefert,
d) Bestimmung von mindestens einer Metrik, die die Energiedichte der Intensitätsverteilung im 3-dimensionalen Raum eines Lichtsbündels durch das Auge und optische Korrektur umfassenden optischen Systems charakterisiert für einen der Parametersätze und die refraktiven Eigenschaften des Auges,
e) Durchführung des Schrittes d) für die Parametersätze, die zur Bestimmung des Zielparametersatzes nach dem in Schritt c) bereitgestellten Verfahren erforderlich sind,
f) Auswahl des Zielparametersatzes, der die Zielgesamtmetrik liefert, aus den Parametersätzen für die der Schritt d) durchgeführt wurde,
g) Bestimmung der erforderlichen optischen Korrektur (2) unter Berücksichtigung des in Schritt f) ausgewählten Zielparametersatzes.

4. Verfahren nach Anspruch 2, **dadurch gekennzeichnet, dass** die Untermetrik in einem Vorzugspropagationsstadium (7) stärker gewichtet wird als die Untermetriken in den Propagationsstadien (9, 10) vor und/oder hinter dem Vorzugspropagationsstadium (7).

5. Verfahren nach Anspruch 4, **dadurch gekennzeichnet, dass** die Untermetriken in den Propagationsstadien (9, 10) vor und/oder hinter dem Vorzugspropagationsstadium (7) mit zunehmendem Abstand vom Vorzugspropagationsstadium (7) geringer gewichtet werden.

6. Verfahren nach einem der Ansprüche 2 bis 5, **dadurch gekennzeichnet, dass** die Zielgesamtmetrik die Gesamtmetrik mit dem maximalen Wert oder einem von diesem geringfügig abweichenden Wert ist.

7. Verfahren nach einem der Ansprüche 2 bis 6, **gekennzeichnet durch** folgende weitere Verfahrensschritte
i) Durchführen der Verfahrensschritte a) bis h) für verschiedene Akkommodationszustände des Auges (1),
j) Auswahl des finalen Zielparametersatzes, der die finale Zielgesamtmetrik liefert aus allen in Schritt i) bestimmten Zielgesamtmetriken für die unterschiedlichen Akkommodationszustände des Auges (1),
k) Bestimmung der erforderlichen optischen Korrektur unter Berücksichtigung des in Schritt j) ausgewählten finalen Zielparametersatzes.

8. Anordnung zur Bestimmung der erforderlichen optischen Korrektur (2) der Fehlsichtigkeit eines Auges (1), welche rechnerisch die optische Korrektur (2) innerhalb eines Zielraums variiert,
**dadurch gekennzeichnet, dass**
eine Analyseeinrichtung vorgesehen ist, welche die Kaustik, nämlich die Einschnürung eines Strahlenbündels, die anstelle eines Bildpunkts entsteht, welche das von einem Objektpunkt ausgehende Strahlenbündel infolge von Abbildungsfehlern zeigt, bevor es wieder auseinander läuft, eines die optische Korrektur und das Auge transmittierenden Lichtstrahls in der räumlichen Umgebung der Netzhaut des Auges mit einer Sollkaustik vergleicht, wobei die optische Korrektur so lange variiert wird, bis ein Abbruchkriterium erfüllt ist, und welche die optische Korrektur (2) innerhalb des Zielraums als erforderliche optische Korrektur (2) auswählt, bei der die Kaustik eines durch die optische Korrektur (2) und das Auge (1) hindurch tretenden Lichtstrahls (3) im Bereich der Netzhaut (6) des Auges (1) vorgegebenen Anforderungen genügt, indem sie die Sollkaustik erreicht oder der Sollkaustik sehr nahe kommt.

9. Anordnung zur Bestimmung der erforderlichen optischen Korrektur (2) der Fehlsichtigkeit eines Auges (1), nach Anspruch 8, mit
- einer Eingabeeinrichtung zur Bereitstellung der refraktiven Eigenschaften des Auges (1),
- einer Analyseeinrichtung, welche
i) mindestens zwei Untermetriken in verschiedenen zugeordneten Propagationsstadien (7, 9, 10) eines Lichtstrahls (3) durch das Auge (1) und optische Korrektur (2) umfassende optische System für einen Parametersatz der die optische Korrektur (2) beschreibenden Größen und die refraktiven Eigenschaften des Auges bestimmt, wobei die Propagationsstadien dadurch festgelegt sind, wie weit ein Lichtstrahl durch das optische System propagiert ist,
ii) aus einer gewichteten Summe der Untermetriken eine Gesamtmetrik bestimmt,
iii) die Schritte i) und ii) für weitere Parametersätze innerhalb eines Suchraums der die optische Korrektur (2) beschreibenden Größen wiederholt, die zur Bestimmung eines Zielparametersatzes erforderlich sind,
iv) den Zielparametersatz aus den Parametersätzen für die die Schritte i) und ii) durchgeführt wurden, auswählt, der die Zielgesamtmetrik liefert, und
v) die erforderliche optische Korrektur (2) unter Berücksichtigung des in Schritt iv) ausgewählten Zielparametersatzes bestimmt.

10. Anordnung zur Bestimmung der erforderlichen optischen Korrektur (2) der Fehlsichtigkeit eines Auges (1), nach Anspruch 8, mit
- einer Eingabeeinrichtung zur Bereitstellung der refraktiven Eigenschaften des Auges (1)
- einer Analyseeinrichtung, welche
i) Größen von die optische Korrektur (2) beschreibenden Parametersätzen innerhalb eines Suchraums bereitstellt,
ii) ein Verfahren zur Bestimmung eines Zielparametersatzes aus den die optische Korrektur (2) beschreibenden Parametersätzen bereitstellt, der für die in Schritt i) bereitgestellten refraktiven Eigenschaften des Auges (1) eine optische Zielkorrektur (2) liefert,
iii) mindestens eine Metrik bestimmt, die die Energiedichte der Intensitätsverteilung im 3- dimensionalen Raum eines Lichtbündels durch das Auge (1) und optische Korrektur (2) umfassenden optischen Systems charakterisiert für einen der Parametersätze und die refraktiven Eigenschaften des Auges,
iv) Schritt iii) für die Parametersätze durchführt, die zur Bestimmung des Zielparametersatzes nach dem in Schnitt ii) bereitgestellten Verfahren erforderlich sind,
v) den Zielparametersatz auswählt, der die Zielmetrik liefert, aus den Parametersätzen für die der Schritt iv) durchgeführt wurde,
vi) die erforderliche optische Korrektur (2) unter Berücksichtigung des in Schritt v) ausgewählten Zielparametersatzes bestimmt.

11. Anordnung nach Anspruch 9 oder 10, **dadurch gekennzeichnet, dass** die Analyse in der Analyseeinrichtung nah oder fern dem Ort der Datenaufnahme stattfindet.

12. Anordnung nach einem der Ansprüche 9, 10 oder 11, **dadurch gekennzeichnet, dass** die Eingabeeinrichtung mit einer Wellenfrontmesseinrichtung zur Messung der refraktiven Eigenschaften des Auges (1) und/oder mit einem Aberrometer nach dem Shack-Hartmann-Prinzip und/oder mit einem Tomographen für das Auge und/oder mit einem Aberrometer für das Tscherning-Verfahren und/oder mit einem Aberrometer nach der Ray-Tracing-Methode verbunden ist.

13. Computerprogrammprodukt mit Programmcodemitteln, die auf einem computerlesbaren Datenträger gespeichert sind, um alle Schritte des Verfahrens nach einem der Ansprüche 1 bis 7 durchzuführen, wenn das Programmprodukt auf einem Computer ausgeführt wird.

14. Computerprogrammprodukt nach Anspruch 13, welches über das Internet oder vergleichbare Netze die Analyse der Daten unabhängig vom Ort der Datenaufnahme an einem beliebigen Ort vornimmt.

15. Datenträger auf dem ein Computerprogramm mit Programmcodemitteln gespeichert ist, um alle Schritte des Verfahrens nach einem der Ansprüche 1 bis 7 auszuführen.

## Claims

1. Method for determining the required optical correction (2) of the refractive error of an eye (1), in which the optical correction (2) is varied computationally within a target space,
**characterized in that**
the caustic network, that is to say the envelope of a bundle of rays, which is produced instead of an image point, which caustic network shows the bundle of rays emanating from an object point as a result of aberrations before said bundle of rays spreads apart again, of a light beam transmitting the optical correction and the eye in the spatial surroundings of the retina of the eye is compared to an intended caustic network, wherein the optical correction is varied until a termination criterion is satisfied, and **in that** the optical correction (2) within the target space is selected as required optical correction (2), in which the caustic network (8) of a light ray passing through the optical correction (2) and the eye (1) meets requirements predetermined in the region of the retina (6) of the eye (1) by virtue of achieving the intended caustic network or coming very close to the intended caustic network.

2. Method for determining the required optical correction (2) of the refractive error of an eye (1) according to Claim 1, comprising the following method steps:
a) providing the refractive properties of the eye (1),
b) providing within a search space variables of parameter sets describing the optical correction (2),
c) providing a method for determining a target parameter set from the parameter sets describing the optical correction (2), which target parameter set supplies the optical target correction (2) for the refractive properties of the eye (1) provided in step a),
d) determining for one of the parameter sets and the refractive properties of the eye at least two sub-metrics in various assigned propagation states (7, 9, 10) of a light ray (3) through the optical system comprising eye and correction, wherein the propagation states are set by virtue of how far a light ray has propagated through the optical system,
e) determining an overall metric from a weighted sum of the sub-metrics,
f) carrying out steps d) and e) for the parameter sets required for determining the target parameter set according to the method provided in step c),
g) selecting the target parameter set which supplies the target overall metric from the parameter sets for which steps d) and e) were carried out,
h) determining the required optical correction (2) taking into account the target parameter set selected in step g).

3. Method for determining the required optical correction (2) of the refractive error of an eye (1) according to Claim 1, comprising the following method steps:
a) providing the refractive properties of the eye (1),
b) providing within a search space variables of parameter sets describing the optical correction (2),
c) providing a method for determining a target parameter set from the parameter sets describing the optical correction (2), which target parameter set supplies an optical target correction (2) for the refractive properties of the eye (1) provided in step a),
d) determining for one of the parameter sets and the refractive properties of the eye at least one metric which characterizes the energy density of the intensity distribution in the 3-dimensional space of a bundle of light through the optical system comprising eye and optical correction,
e) carrying out step d) for the parameter sets required for determining the target parameter set according to the method provided in step c),
f) selecting the target parameter set which supplies the target overall metric from the parameter sets for which step d) was carried out,
g) determining the required optical correction (2) taking into account the target parameter set selected in step f).

4. Method according to Claim 2, **characterized in that** the sub-metric in a preferred propagation state (7) is weighted more strongly than the sub-metrics in the propagation states (9, 10) upstream and/or downstream of the preferred propagation state (7).

5. Method according to Claim 4, **characterized in that** the sub-metrics in the propagation states (9, 10) upstream and/or downstream of the preferred propagation state (7) are weighted less strongly with increasing distance from the preferred propagation state (7).

6. Method according to one of Claims 2 to 5, **characterized in that** the target overall metric is the overall metric with the maximum value or a value slightly deviating therefrom.

7. Method according to one of Claims 2 to 6, **characterized by** the following further method steps:
i) carrying out method steps a) to h) for various accommodation states of the eye (1),
j) selecting the final target parameter set, which supplies the final target overall metric from all target overall metrics determined in step i) for the different accommodation states of the eye (1),
k) determining the required optical correction taking into account the final target parameter set selected in step j).

8. Arrangement for determining the required optical correction (2) of the refractive error of an eye (1), which arrangement varies the optical correction (2) computationally within a target space,
**characterized in that**
provision is made of an analysis apparatus, which compares the caustic network, that is to say the envelope of a bundle of rays, which is produced instead of an image point, which caustic network shows the bundle of rays emanating from an object point as a result of aberrations before said bundle of rays spreads apart again, of a light beam transmitting the optical correction and the eye in the spatial surroundings of the retina of the eye to an intended caustic network, wherein the optical correction is varied until a termination criterion is satisfied, and which analysis apparatus selects the optical correction (2) within the target space as required optical correction (2), in which the caustic network of a light ray (3) passing through the optical correction (2) and the eye (1) meets requirements predetermined in the region of the retina (6) of the eye (1) by virtue of achieving the intended caustic network or coming very close to the intended caustic network.

9. Arrangement for determining the required optical correction (2) of the refractive error of an eye (1) according to Claim 8, comprising:
- an input apparatus for providing the refractive properties of the eye (1),
- an analysis apparatus, which
i) determines for one parameter set of the variables describing the optical correction (2) and the refractive properties of the eye at least two sub-metrics in various assigned propagation states (7, 9, 10) of a light ray (3) through the optical system comprising eye (1) and optical correction (2), wherein the propagation states are set by virtue of how far a light ray has propagated through the optical system,
ii) determines an overall metric from a weighted sum of the sub-metrics,
iii) repeats steps i) and ii) for further parameter sets within a search space of the variables describing the optical correction (2), which variables are required for determining a target parameter set,
iv) selects the target parameter set which supplies the target overall metric from the parameter sets for which steps i) and ii) were carried out, and
v) determines the required optical correction (2) taking into account the target parameter set selected in step iv).

10. Arrangement for determining the required optical correction (2) of the refractive error of an eye (1) according to Claim 8, comprising:
- an input apparatus for providing the refractive properties of the eye (1),
- an analysis apparatus, which
i) provides within a search space variables of parameter sets describing the optical correction (2),
ii) provides a method for determining a target parameter set from the parameter sets describing the optical correction (2), which target parameter set supplies an optical target correction (2) for the refractive properties of the eye (1) provided in step i),
iii) determines for one of the parameter sets and the refractive properties of the eye at least one metric which characterizes the energy density of the intensity distribution in the 3-dimensional space of a bundle of light through the optical system comprising eye (1) and optical correction (2),
iv) carries out step iii) for the parameter sets required for determining the target parameter set according to the method provided in step ii),
v) selects the target parameter set which supplies the target metric from the parameter sets for which step iv) was carried out,
vi) determines the required optical correction (2) taking into account the target parameter set selected in step v).

11. Arrangement according to Claim 9 or 10, **characterized in that** the analysis in the analysis apparatus takes place close to, or far away from, the location where the data is recorded.

12. Arrangement according to one of Claims 9, 10 or 11, **characterized in that** the input apparatus is connected to a wavefront measurement apparatus for measuring the refractive properties of the eye (1) and/or to an aberrometer operating according to the Shack/Hartmann principle and/or to a tomography scanner for the eye and/or to an aberrometer for the Tscherning method and/or to an aberrometer for the ray tracing method.

13. Computer program product with program code means stored on a computer-readable data medium for carrying out all steps of the method according to one of Claims 1 to 7 when the program product is executed on a computer.

14. Computer program product according to Claim 13, which undertakes the analysis of the data at any location via the Internet or comparable networks, independently of the location where the data is recorded.

15. Data medium on which a computer program with program code means is stored for carrying out all steps of the method according to one of Claims 1 to 7.

## Revendications

1. Procédé destiné à déterminer la correction (2) optique requise pour le défaut de vision d'un oeil (1), lors duquel on fait varier la correction (2) optique par calcul au sein d'une plage cible,
**caractérisé en ce**
**qu'**on compare le plan focal, à savoir le rétrécissement d'un faisceau de rayons qui se produit à la place d'un pixel, lequel plan focal montre le faisceau de rayons naissant à partir d'un point sur l'objet suite à des défauts de reproduction, avant qu'il ne se repartage, d'un faisceau lumineux transmettant la correction optique et l'oeil dans l'environnement spatial de la rétine de l'oeil avec un plan focal de consigne, la correction optique étant variée jusqu'à ce qu'un critère d'interruption soit satisfait, et en ce qu'on choisit la correction (2) optique à l'intérieur de la plage cible comme étant la correction (2) optique requise, avec laquelle le plan focal (8) d'un faisceau lumineux traversant la correction (2) optique et l'oeil (1) satisfait à des exigences prédéfinies dans la région de la rétine (6) de l'oeil (1) en atteignant le plan focal de consigne ou en s'approchant fortement du plan focal de consigne.

2. Procédé destiné à déterminer la correction (2) optique requise pour le défaut de vision d'un oeil (1) selon la revendication 1, avec les étapes de procédé suivantes :
a) mise à disposition des caractéristiques réfractives de l'oeil (1),
b) mise à disposition de grandeurs de jeux de paramètres décrivant la correction (2) optique dans une plage de recherche,
c) mise à disposition d'un procédé destiné à déterminer un jeu de paramètres cibles parmi les jeux de paramètres décrivant la correction (2) optique qui fournit une correction (2) optique cible pour les caractéristiques réfractives de l'oeil (1) mises à disposition lors de l'étape a),
d) détermination d'au moins deux sous-métriques à différents stades de propagation (7, 9, 10) associés d'un faisceau lumineux (3) à travers l'oeil et le système optique comprenant la correction pour l'un des jeux de paramètres et les caractéristiques réfractives de l'oeil, les stades de propagation étant fixés par la distance à laquelle un faisceau lumineux est propagé à travers le système optique,
e) détermination d'une métrique totale à partir d'une somme pondérée des sous-métriques,
f) réalisation des étapes d) et e) pour les jeux de paramètres qui sont requis pour déterminer le jeu de paramètres cibles selon le procédé mis à disposition dans l'étape c),
g) choix du jeu de paramètres cibles qui fournit la métrique totale cible parmi les jeux de paramètres pour lesquels les étapes d) et e) ont été réalisées,
h) détermination de la correction (2) optique requise, en prenant en considération le jeu de paramètres cibles choisi dans l'étape g).

3. Procédé destiné à déterminer la correction (2) optique requise pour le défaut de vision d'un oeil (1) selon la revendication 1, avec les étapes de procédé suivantes :
a) mise à disposition des caractéristiques réfractives de l'oeil (1),
b) mise à disposition de grandeurs de jeux de paramètres décrivant la correction (2) optique dans une plage de recherche,
c) mise à disposition d'un procédé destiné à déterminer un jeu de paramètres cibles parmi les jeux de paramètres décrivant la correction (2) optique qui fournit une correction (2) optique cible pour les caractéristiques réfractives de l'oeil (1) mises à disposition lors de l'étape a),
d) détermination d'au moins une métrique qui caractérise la densité d'énergie de la distribution d'intensité dans l'espace tridimensionnel d'un faisceau lumineux à travers l'oeil et d'un système optique comprenant la correction optique pour l'un des jeux de paramètres et les caractéristiques réfractives de l'oeil,
e) réalisation de l'étape d) pour les jeux de paramètres qui sont requis pour déterminer le jeu de paramètres cibles selon le procédé mis à disposition dans l'étape c),
f) choix du jeu de paramètres cibles qui fournit la métrique totale cible parmi les jeux de paramètres pour lesquels l'étape d) a été réalisée,
g) détermination de la correction (2) optique requise, en prenant en considération le jeu de paramètres cibles choisi dans l'étape f).

4. Procédé selon la revendication 2, **caractérisé en ce que** la sous-métrique dans un stade de propagation (7) préférentiel est pondérée plus fortement que les sous-métriques dans les stades de propagation (9, 10) avant et/ou après le stade de propagation (7) préférentiel.

5. Procédé selon la revendication 4, **caractérisé en ce que** les sous-métriques dans les stades de propagation (9, 10) avant et/ou après le stade de propagation (7) préférentiel sont plus faiblement pondérées à mesure que l'écart par rapport au stade de propagation (7) préférentiel augmente.

6. Procédé selon l'une quelconque des revendications 2 à 5, **caractérisé en ce que** la métrique totale cible est la métrique totale avec la valeur maximale ou avec une valeur faiblement divergente de celle-ci.

7. Procédé selon l'une quelconque des revendications 2 à 6, **caractérisé par** les étapes de procédé supplémentaires suivantes :
i) réalisation des étapes de procédé a) à h) pour différents états d'accommodation de l'oeil (1),
j) choix du jeu final de paramètres cibles qui fournit la métrique cible totale finale parmi toutes les métriques cibles totales déterminées dans l'étape i) pour les différents états d'accommodation de l'oeil (1),
k) détermination de la correction optique requise, en prenant en considération le jeu final de paramètres cibles choisi dans l'étape j).

8. Agencement destiné à déterminer la correction (2) optique requise pour le défaut de vision d'un oeil (1), lequel fait varier la correction optique (2) par calcul au sein d'une plage cible,
**caractérisé en ce**
**qu'**il est prévu un dispositif d'analyse, lequel compare le plan focal, à savoir le rétrécissement d'un faisceau de rayons qui se produit à la place d'un pixel, lequel plan focal montre le faisceau de rayons naissant à partir d'un point sur l'objet suite à des défauts de reproduction, avant qu'il ne se repartage, d'un faisceau lumineux transmettant la correction optique et l'oeil dans l'environnement spatial de la rétine de l'oeil avec un plan focal de consigne, la correction optique étant variée jusqu'à ce qu'un critère d'interruption soit satisfait, et lequel choisit la correction (2) optique à l'intérieur de la plage cible comme étant la correction (2) optique requise, avec laquelle le plan focal d'un faisceau lumineux (3) traversant la correction (2) optique et l'oeil (1) satisfait à des exigences prédéfinies dans la région de la rétine (6) de l'oeil (1) en atteignant le plan focal de consigne ou en s'approchant fortement du plan focal de consigne.

9. Agencement destiné à déterminer la correction (2) optique requise pour le défaut de vision d'un oeil (1) selon la revendication 8, avec
- un dispositif de saisie pour mettre à disposition les caractéristiques réfractives de l'oeil (1),
- un dispositif d'analyse, lequel
i) détermine au moins deux sous-métriques à différents stades de propagation (7, 9, 10) associés d'un faisceau lumineux (3) à travers l'oeil (1) et le système optique comprenant la correction (2) optique pour un jeu de paramètres des grandeurs décrivant la correction (2) optique et les caractéristiques réfractives de l'oeil, les stades de propagation étant fixés par la distance à laquelle un faisceau lumineux est propagé à travers le système optique,
ii) détermine une métrique totale à partir d'une somme pondérée des sous-métriques,
iii) répète les étapes i) et ii) pour des jeux de paramètres supplémentaires à l'intérieur d'une plage de recherche des grandeurs décrivant la correction (2) optique, lesquelles sont requises pour la détermination d'un jeu de paramètres cibles,
iv) choisit le jeu de paramètres cibles qui fournit la métrique totale cible parmi les jeux de paramètres pour lesquels les étapes i) et ii) ont été réalisées, et
v) détermine la correction (2) optique requise, en prenant en considération le jeu de paramètres cibles choisi dans l'étape iv).

10. Agencement destiné à déterminer la correction (2) optique requise pour le défaut de vision d'un oeil (1) selon la revendication 8, avec
- un dispositif de saisie pour mettre à disposition les caractéristiques réfractives de l'oeil (1),
- un dispositif d'analyse, lequel
i) met à disposition des grandeurs de jeux de paramètres décrivant la correction (2) optique dans une plage de recherche,
ii) met à disposition un procédé destiné à déterminer un jeu de paramètres cibles parmi les jeux de paramètres décrivant la correction (2) optique qui fournit une correction (2) optique cible pour les caractéristiques réfractives de l'oeil (1) mises à disposition dans l'étape i),
iii) détermine au moins une métrique qui caractérise la densité d'énergie de la distribution d'intensité dans l'espace tridimensionnel d'un faisceau lumineux à travers l'oeil (1) et d'un système optique comprenant la correction (2) optique pour l'un des jeux de paramètres et les caractéristiques réfractives de l'oeil,
iv) réalise l'étape iii) pour les jeux de paramètres qui sont requis pour déterminer le jeu de paramètres cibles selon le procédé mis à disposition dans l'étape ii),
v) choisit le jeu de paramètres cibles qui fournit la métrique cible parmi les jeux de paramètres pour lesquels l'étape iv) a été réalisée,
vi) détermine la correction (2) optique requise, en prenant en considération le jeu de paramètres cibles choisi dans l'étape v).

11. Agencement selon la revendication 9 ou 10, **caractérisé en ce que** l'analyse dans le dispositif d'analyse est effectuée à proximité ou à distance du lieu de l'enregistrement des données.

12. Agencement selon l'une quelconque des revendications 9, 10 ou 11, **caractérisé en ce que** le dispositif de saisie est relié à un dispositif de mesure du front d'ondes, destiné à mesurer les caractéristiques réfractives de l'oeil (1) et/ou à un aberromètre selon le principe de Shack-Hartmann et/ou à un tomographe pour l'oeil et/ou à un aberromètre pour le procédé de Tscherning et/ou à un aberromètre selon la méthode du ray tracing (lancer de rayon).

13. Produit sous forme de programme informatique, avec des moyens de code programme qui sont mémorisés sur un support de données lisible par ordinateur, pour réaliser toutes les étapes du procédé selon l'une quelconque des revendications 1 à 7, lorsque le produit sous forme de programme est exécuté sur un ordinateur.

14. Produit sous forme de programme informatique selon la revendication 13, lequel procède via Internet ou des réseaux comparables à l'analyse des données, indépendamment du lieu de l'enregistrement des données en un lieu quelconque.

15. Support informatique sur lequel est mémorisé un programme informatique avec des moyens de code programme, pour réaliser toutes les étapes du procédé selon l'une quelconque des revendications 1 à 7.
